Europäisches Patentamt

European Patent Office

Office européen des brevets

(19)

(11) Veröffentlichungsnummer: **0 279 293**
**A2**

## (12) EUROPÄISCHE PATENTANMELDUNG

(21) Anmeldenummer: 88101636.4

(22) Anmeldetag: 04.02.88

(51) Int. Cl.⁴: **A61B 6/14**

(30) Priorität: 16.02.87 DE 3704857

(43) Veröffentlichungstag der Anmeldung:
24.08.88 Patentblatt 88/34

(84) Benannte Vertragsstaaten:
**DE FR GB IT SE**

(71) Anmelder: **Siemens Aktiengesellschaft Berlin und München**
**Wittelsbacherplatz 2**
**D-8000 München 2(DE)**

(72) Erfinder: **Günther, Werner**
**Odenwaldstrasse 20**
**D-6140 Bensheim(DE)**
Erfinder: **Müther, Manfred**
**Adolf-Kolping-Strasse 20**
**D-6140 Bensheim(DE)**
Erfinder: **Heubeck, Erich**
**Blütenweg 11**
**D-6140 Bensheim(DE)**
Erfinder: **Döbert, Michael**
**Zedernstrasse 2**
**D-6143 Lorsch(DE)**
Erfinder: **Werner, Leonard, Dipl.-Ing.**
**Gleiwitzer Strasse 3**
**D-6944 Hemsbach(DE)**

(54) **Zahnärztliche Röntgendiagnostikeinrichtung zur Erstellung von Panorama-Schichtaufnahmen vom Kiefer eines Patienten.**

(57) Die Einrichtung enthält eine Dreheinheit (1), die Träger einerseits einer Röntgenstrahlenquelle (2) und andererseits einer Blende (4) mit Sekundärspalt (7) sowie einer Detektoranordnng (5) ist, welche elektrische Signale proportional zur Strahlungsintensität bildet. Zur Vereinfachung insbesondere der Signalaufbereitung ist erfindungsgemäß die Detektoranordnung (5) aus ein oder mehreren, der Größe des Sekundärspaltes (7) entsprechenden Halbleiterdetektoren mit Szintillationsschicht gebildet. Die von der Detektoranordnung gewonnenen Spannungen werden digitalisiert und anschließend einem Bildspeicher (10) zugeführt. Eine Datenverarbeitungseinrichtung mit Rechner (13) berechnet aus den von der Detektoranordnung während eines Aufnahmeablaufs gelieferten Signalen ein Übersichtsbild. Dabei werden die Signale aus dem Bildspeicher (10) zeitgerecht, d.h. entsprechend dem Schichtbild einer gewünschten Schichtlage, aufaddiert.

FIG 2

EP 0 279 293 A2

## Zahnärztliche Röntgendiagnostikeinrichtung zur Erstellung von Panorama-Schichtaufnahmen vom Kiefer eines Patienten

Die Erfindung bezieht sich auf eine zahnärztliche Röntgendiagnostikeinrichtung zur Erstellung von Panorama-Schichtaufnahmen vom Kiefer eines Patienten, enthaltend eine um eine vertikale Achse drehbare Einheit, die Träger einerseits einer Röntgenstrahlenquelle und andererseits einer Blende mit Sekundärspalt und einer Detektoranordnung ist, welche elektrische Signale proportional zur Strahlungsintensität bildet, enthaltend ferner einen mit der Detektoranordnung verbundenen A/D-Wandler, der die von der Detektoranordnung gewonnenen Spannungen digitalisiert, einen Bildspeicher sowie eine Datenverarbeitungseinrichtung mit Rechner, welche aus den von der Detektoranordnung während eines Aufnahmeablaufs gelieferten Signalen ein Übersichtsbild berechnet und eine an die Datenverarbeitungseinrichtung angeschlossene Bildwiedergabeeinrichtung.

Eine Einrichtung dieser Art ist beispielsweise aus der DE-PS 26 46 638 bekannt. Gegenüber konventioneller Aufnahmetechnik mit Röntgenfilm kann bei dieser bekannten Einrichtung eine Panorama-Röntgenaufnahme anstelle auf einem Röntgenfilm elektronisch aufgezeichnet und abgespeichert und - schließlich auf einem Monitor als Darstellungseinrichtung wiedergegeben werden. Bei der bekannten Einrichtung ist der zur Anwendung vorgesehene Detektor nicht weiter beschrieben.

Der im Patentanspruch 1 angegebenen Erfindung liegt die Aufgabe zugrunde, eine gegenüber dem Stand der Technik verbesserte zahnärztliche Röntgendiagnostikeinrichtung anzugeben, die insbesondere hinsichtlich des Bildaufnehmers und der Aufbereitung der Signale vereinfacht ist.

Die gemäß der Erfindung vorgesehene Detektoranordnung kann aus ein oder mehreren der Größe des Sekundärspaltes entsprechenden Einzeldetektoren bestehen; vorteilhafterweise sind diese auf der Basis von amorphem Silizium (a Si:H) gebildet und so gestaltet, daß sie eine vergleichsweise kurze Auslesezeit von beispielsweise weniger als 5 ms pro Spaltbild haben.

Die gemäß einer vorteilhaften Weiterbildung der Erfindung vorgesehene Vorverarbeitungseinheit kann vorteilhafterweise so ausgebildet sein, daß nicht alle Informationen, sondern nur diejenigen einiger benachbarter Spalte, zeitgerecht addiert werden, d.h. so addiert, daß ein Schichtbild einer gewünschten Schichtlage entsteht. Sofern der vorgesehene Bildspeicher bei vertretbarer Baugröße und vertretbaren Kosten eine entsprechend große Kapazität für die aufzunehmenden Daten aufweist, kann die Vorverarbeitungseinheit entbehrlich sein.

Nachfolgend wird ein Ausführungsbeispiel der Erfindung näher erläutert. Es zeigen:

Figur 1 ein Prinzipschaltbild einer Röntgendiagnostikeinrichtung nach der Erfindung,

Figur 2 ein Blockschaltbild für die Signalaufbereitung.

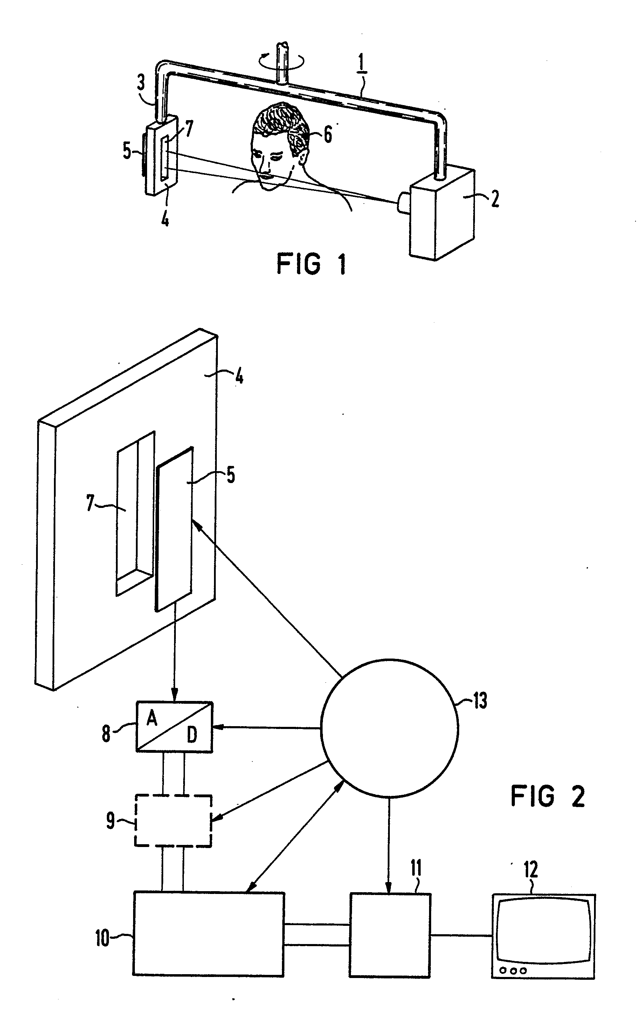

Die Figur 1 zeigt in einer Prinzipdarstellung eine zahnärztliche Röntgendiagnostikeinrichtung, die eine Dreheinheit 1 enthält, an der einerseits eine Röntgenstrahlenquelle 2 und andererseits ein Träger 3 für eine Sekundärblende 4 und eine Detektoranordnung 5 angeordnet sind. Die Dreheinheit 1 kann in bekannter Weise, wie durch Pfeil angegeben, um den Patienten 6 herumgedreht werden. Die hierzu erforderliche Verstell und Steuereinrichtungen sind hinreichend bekannt, so daß deren Erläuterung entbehrlich ist. Es sei an dieser Stelle lediglich vermerkt, daß der Kopf des Patienten während einer Aufnahme mittels einer Halterung fixiert ist, die Dreheinheit sich bei einer vorgegebenen Aufnahmezeit mit einer bestimmten Geschwindigkeit um den Patienten bewegt und die Anordnung so getroffen ist, daß die Röntgenstrahlen stets im wesentlichen rechtwinkelig auf den aufzunehmenden Kiefer treffen und ein etwa gleichbleibender Abstand Kiefer-Detektoranordnung gegeben ist.

Wie auch in Figur 2 angedeutet, enthält die Sekundärblende 4 einen Sekundärspalt 7 in der Größe von beispielsweise 5 $\times$ 125 mm. Die Detektoranordnung 5 besteht aus einem einzigen Halbleiterbildaufnehmer mit aufgebrachter Szintillationsschicht. Die Szintillatorschicht kann direkt oder über eine faseroptische Platte mit dem Bildaufnehmer gekoppelt sein. Zweckmäßigerweise weist der Bildaufnehmer zumindest die Maße des Sekundärspaltes 7 auf. Der Bildaufnehmer ist auf der Basis von Fotodiodenarrays aus amorphem Silizium (a Si:H) aufgebaut. Die Pixelgröße ist, um eine sinnvolle Auflösung zu bekommen, vorteilhafterweise kleiner als 0,3 mm x 0,3 mm. Um die Verwischungseffekte möglichst klein zu halten, hat der Detektor eine Auslesezeit von kleiner als 5 ms; das entspricht einer Auslesezeit von < 1 μsec pro Pixel.

Die Detektoranordnung 5 ist mit einem A/D-Wandler 8 verbunden, an den sich ein digitales Bildverarbeitungssystem mit einer Vorverarbeitungseinheit 9, einem Bildspeicher 10, einer Bildausleseeinheit 11, einem Monitor 12 und einem Rechner 13 anschließen.

Am Ausgang der Detektoranordnung 5 liegen Bildinformationen in Form von Spannungen an, die der Röntgenstrahlungsintensität in dem betreffenden Pixel des Detektors proportional sind.

Die anliegenden Spannungen werden im A/D-Wandler digitalisiert; die einzelnen Werte können sodann entweder direkt im Bildspeicher 10 oder nach Vorverarbeitung in der Einheit 9 im Bildspeicher 10 abgespeichert werden. Der Rechner 13 gibt hierzu die notwendigen Steuer-bzw. Auslesebefehle.

Die direkte Eingabe der vom A/D-Wandler erhaltenen Signale in den Bildspeicher 10 ist dann vorteilhaft, wenn dieser bei vertretbarem Kostenaufwand bzw. Bauvolumen eine entsprechend große Kapazität aufweist. In diesem Falle werden die Daten während des Umlaufs zunächst nur abgespeichert und erst nach Abschluß der Aufnahme, also nach Umlauf des Strahlers um den Patientenkopf, verarbeitet; d.h. so addiert, daß ein Schichtbild der gewünschten Schichtlage entsteht. Wenngleich hierzu eine relativ große Datenmenge verarbeitet werden muß, so hat diese Lösung den Vorteil, daß eine nachträgliche Darstellung mehrerer verschiedener Schichten möglich ist.

Ist die unter vorgenanntem Gesichtspunkt notwendige Bildspeicherkapazität nicht mit wirtschaftlich vertretbarem Aufwand zu erhalten, so kann es vorteilhaft sein, die in Fig. 2 gestrichelt angegebene Vorverarbeitungseinheit 9 zwischenzuschalten, die einen Zwischenspeicher und einen Signalprozessor enthält, wodurch die digitalen Daten aus dem A/D-Wandler entsprechend einem Steuerbefehl aus dem Rechner 13 zeitgerecht addiert, d.h. so addiert werden, daß ein Schichtbild einer gewünschten Schichtlage entsteht. Die hieraus resultierenden aufgearbeiteten Daten werden anschließend in den Bildspeicher gegeben. Durch die Zwischenschaltung der Vorverarbeitungseinheit 9 kann also ein Bildspeicher mit kleinerer Kapazität verwendet werden. Allerdings ist die Schichtlage durch Zwischenschalten einer solchen Vorverarbeitungseinheit festgelegt, d.h. die Schichtlage kann nicht mehr in gewissen Grenzen verändert werden, im Gegensatz zu der vorerwähnten Anordnung ohne Vorverarbeitungseinheit 9, wo die Bilddaten erst nach einer Aufnahme zu einem Schichtbild zusammengesetzt werden.

Zwischen beiden Möglichkeiten kann auch ein Kompromiß vorgesehen sein, gemäß dem vor der Abspeicherung mehrere benachbarte Bildspalten zeitgerecht aufaddiert werden und die Addition dieser Summenspalten zu einer Bildspalte erst nach der Abspeicherung erfolgt. Zu diesem Zweck kann die Vorverarbeitungseinheit so ausgebildet sein, daß nicht alle Informationen, sondern nur diejenigen von einigen benachbarten Spalten, zeitgerecht im Rechner addiert und sodann in den Bildspeicher gegeben werden.

Zur Bildentstehung sei erläuternd auf folgendes hingewiesen:

Eine Bildaufzeichnung kommt dadurch zustande, daß bei eingeschaltetem Strahler und sich drehender Einheit bei jedem Winkelgrad vom Detektor Signale entstehen, die der Strahlungsintensität der den Patientenkopf durchdringenden und durch den Sekundärschlitz begrenzten Strahlung proportional sind.

Die durch den Sekundärschlitz hindurchgehende Strahlungsenergie ändert sich ständig, wenn der Röntgenstrahler langsam um den Kopf des Patienten herumläuft, d.h. je dichter das Objekt für die Strahlung ist (Knochen und Zähnen im Vergleich zu Weichteilen), umso größer ist die Menge der von dem dichteren Objekt absorbierten Röntgenstrahlung, wobei auch eine entsprechend geringere Menge an Röntgenstrahlen den Detektor erreicht, wodurch ein weniger dichtes Bild des Objektes an dieser Stelle erscheint. Wenn die augenblickliche Strahlungsenergie am Spalt in ein Bild umgewandelt würde, würde dies einem Bild entsprechen, das im wesentlichen die gleichen Dimensionen wie der Spalt haben würde, wobei die Intensität an jedem Punkt des Bildes der Dichte des Objekts entlang der Achse der Röntgenstrahlen an diesem Punkt entspricht. Diese Stabmuster in Form digitaler Werte werden laufend abgespeichert und mit Hilfe des Rechners zeitgerecht addiert und schließlich elektronisch zu einem Gesamtbild zusammengesetzt. Das zeitgerechte Addieren erfolgt dabei so, als ob bei konventioneller Technik der Röntgenfilm bei einem konventionellen zahnärztlichen Panorama-Röntgengerät hinter dem Sekundärspalt in einer Filmkassette entlang bewegt werden würde. Für das zeitgerechte Addieren gilt, wenn v die Filmgeschwindigkeit ist, die in konventioneller Technik zu der gewünschten Schichtlage führt, folgende Beziehung:

$$S_{ni} = \sum_{m=1}^{M} \sigma_{mi}\left(t - m\ \frac{a}{v}\right)$$

$i = 1 \ldots i_{max} \qquad n = 1 \ldots n_{max}$

wobei im einzelnen bedeuten:

$n$     die Spaltennummer im fertigen Bild,

$n_{max}$   die Anzahl Spalten im fertigen Bild,

$i$     die Zeilennummer im fertigen Bild,

$i_{max}$   die Anzahl der Zeilen im fertigen Bild,

$S_{ni}$   der Wert des Pixels in der i-ten Zeile und n-ten Spalte des fertigen Bildes,

M   die Anzahl der Spalten des Bildaufnehmers,

σmi   (t) der Wert des Pixels in der m-ten Spalte und der i-ten Zeile des Bildaufnehmers zum Zeitpunkt t,

a   die Pixelgröße.

Wie an sich bekannt, bestimmt bei herkömmlichen zahnärztlichen Panorama-Röntgengeräten die (nicht konstante) Geschwindigkeit, mit der der Film hinter dem Sekundärschlitz vorbeigeführt wird, die Lage und die Form der Schicht des röntgenografisch erfaßten Objektes. Dies bedeutet, daß durch Änderung der Geschwin digkeit an sich die Lage und die Form der scharf eingestellten Schicht oder Ebene verändert werden kann. Im vorliegenden Anwendungsfall können diese unterschiedlichen Schichtlagen durch unterschiedlich zeitgerechtes Addieren der Daten bestimmt werden.

## Ansprüche

1. Zahnärztliche Röntgendiagnostikeinrichtung zur Erstellung von Panorama-Schichtaufnahmen vom Kiefer eines Patienten, enthaltend eine um eine vertikale Achse drehbare Einheit (1), die Träger einerseits einer Röntgenstrahlenquelle (2) und andererseits einer Blende (4) mit Sekundärspalt (7) und einer Detektoranordnung (5) ist, welche elektrische Signale proportional zur Strahlungsintensität bildet, enthaltend ferner einen mit der Detektoranordnung (5) verbundenen A/D-Wandler (8), der die von der Detektoranordnung gewonnenen Spannungen digitalisiert, einen Bildspeicher (10) sowie eine Datenverarbeitungseinrichtung mit Rechner (13), welche aus den von der Detektoranordnung während eines Aufnahmeablaufs gelieferten Signalen ein Übersichtsbild berechnet und eine an die Datenverarbeitungseinrichtung angeschlossene Bildwiedergabeeinrichtung (12), **dadurch gekennzeichnet**, daß die Detektoranordnung (5) aus ein oder mehreren, der Größe des Sekundärspaltes (7) entspechenden Halbleiterdetektoren mit Szintillationsschicht gebildet ist und daß mit dem Rechner (13) einerseits dem Wandler (8), dem Bildspeicher (10) und einer Bildausleseeinheit (11) Steuerbefehle zugeführt und andererseits die Signale aus dem Bildspeicher (10) zeitgerecht, d.h. entsprechend dem Schichtbild einer gewünschten Schichtlage aufaddiert werden.

2. Röntgendiagnostikeinrichtung nach Anspruch 1, **dadurch gekennzeichnet**, daß Halbleiterdetektoren mit einer Pixelgröße von $\leq 0{,}5 \times 0{,}5$ mm vorgesehen sind.

3. Röntgendiagnostikeinrichtung nach Anspruch 2, **dadurch gekennzeichnet**, daß die Halbleiterdetektoren auf der Basis von amorphem Silizium (a Si:H) gebildet sind.

4. Röntgendiagnostikeinrichttung nach einem der Ansprüche 1 bis 3, **dadurch gekennzeichnet**, daß ein einziger, den Sekundärspalt (7) abdeckender Detektor (5) vorgesehen ist.

5. Röntgendiagnostikeinrichtung nach einem der Ansprüche 1 bis 4, **dadurch gekennzeichnet**, daß zwischen A/D-Wandler (8) und Bildspeicher (10) eine Vorverarbeitungseinheit (9) mit einem Zwischenspeicher und einem Arithmetik-Prozessor, vorgesehen ist, welche die digitalisierten Daten aus dem Wandler (8) entsprechend einem Steuerbefehl aus dem Rechner (13) zeitgerecht addiert und diese Bilddaten anschließend in den Bildspeicher (10) eingibt.

0 279 293

FIG 1

FIG 2